# EUROPÄISCHE PATENTSCHRIFT

(11) **EP 0 615 746 B1**
(45) Veröffentlichungstag und Bekanntmachung des Hinweises auf die Patenterteilung: **07.07.1999**
(21) Anmeldenummer: 94103244.3
(22) Anmeldetag: 04.03.1994
(51) Int. Cl.: A61K 9/127, A61K 7/00

(54) **Wässriges Liposomensystem sowie Verfahren zur Herstellung eines derartigen Liposomensystems**
Aqueous liposomal system and process for preparing such a liposomal system
Système aqueux de liposomes et procédé pour la préparation d'un tel système de liposomes

(30) Priorität: 15.03.1993 DE 4308121
(43) Veröffentlichungstag der Anmeldung: 21.09.1994
(73) Patentinhaber: RHONE-POULENC RORER GMBH, 50792 Köln (DE)
(72) Erfinder: Hager, Jörg-Christian, D-50827 Köln (DE); Löhr, Josef-Peter, Dr., D-40724 Hilden (DE); Dürr, Manfred, Dr., D-50129 Bergheim-Glessen (DE)
(74) Vertreter: Lau-Loskill, Philipp, Dipl.-Phys.

(56) Entgegenhaltungen:
- EP-A- 0 475 160
- WO-A-90/15593
- WO-A-94/01089

## Beschreibung

Die vorliegende Erfindung betrifft ein wäßriges Liposomensystem mit den Merkmalen des Oberbegriffs des Patentanspruchs 1 sowie ein Verfahren zur Herstellung eines derartigen Liposomensystems.

Wäßrige phospholipidische Liposomensysteme sind für verschiedene Anwendungen bekannt. So werden diese Systeme beispielsweise im kosmetischen Bereich oder für die Herstellung von pharmazeutischen Produkten eingesetzt, wobei sich die Systeme dadurch auszeichnen, daß sie kugelförmige Vesikel, die auch als Liposome bezeichnet werden, aufweisen, die in ihrem Inneren eine wäßrige Phase enthalten. Abhängig von dem jeweiligen Anwendungszweck kann dann in der im Inneren der kugelförmigen Vesikel vorgesehenen wäßrigen Phase ein pharmazeutischer oder kosmetischer Wirkstoff gelöst, dispergiert, suspendiert oder emulgiert sein. Als Beispiel für derartige, mit einem Wirkstoff versehene Liposomensysteme sind die Systeme zu benennen, die aus der EP 0 309 519 A oder der EP 0 315 467 A bekannt sind, wobei bei den bekannten Liposomensystemen der Wirkstoff Pentamidin in den Vesikeln eingekapselt ist.

Desweiteren sind auch wäßrige Liposomensysteme bekannt, die im Inneren der Vesikel nur die wäßrige Phase aufweisen, wobei derartige Liposomensysteme auch als Leer-Liposomensysteme bezeichnet werden, die dann ggf. später mit einem pharmazeutischen oder kosmetischen Wirkstoff beladen werden.

Bei den beiden, zuvor beschriebenen wäßrigen Liposomensystemen sind die Liposomen durch eine Lipiddoppelmembran nach außen hin begrenzt.

Ein vesikelhaltiges wäßriges System mit den Merkmalen des Oberbegriffs des Patentanspruchs 1 ist aus der EP 0 475 160 A bekannt. Hierbei weist das bekannte wäßrige System neben dem mindestens einen Phospholipid desweiteren als nicht phospholipidische Substanz Gallensäure, ein Gallensäuresalz, ein Gallensäurederivat oder ein Salz eines Gallensäurederivates auf, wobei jedoch in den Ausführungsbeispielen als kleinstes Verhältnis von Phospholipid zum Natriumcholat von 1:0,107 genannt wird. Ausdrücklich weist die EP 0 475 160 A darauf hin, daß das hierin beschriebene vesikelhaltige wäßrige System kein Liposomensystem sondern ein Transfersomensystem darstellt, wobei sich diese bekannten Transfersomen in mindestens drei Grundeigenschaften, die in der EP 0 475 160 A ausführlich beschrieben sind, von den Liposomen unterscheiden.

Die bekannten Liposomensysteme weisen häufig den Nachteil auf, daß sie die Tendenz besitzen, schon nach kurzer Zeit einen unerwünschten Bodensatz auszubilden.

Der vorliegenden Erfindung liegt die Aufgabe zugrunde, ein vesikelhalhges wäßriges System, das mindestens ein Phospholipid aufweist, zur Verfügung zu stellen, das eine besonders hohe Stabilität besitzt und somit nicht zur Ausbildung eines Bodensatzes neigt.

Diese Aufgabe wird erfindungsgemäß durch ein vesikelhaltiges wäßriges System mit den kennzeichnenden Merkmalen des Patentanspruchs 1 gelöst.

Das erfindungsgemäße vesikelhaltige wäßrige System, das mindestens ein Phospholipid sowie mindestens eine weitere, nicht phospholipidische Substanz enthält, bei der es sich um Gallensäure, ein Gallensäuresalz, ein Gallensäurederivat und/oder um ein Salz eines Gallensäurederivates handelt, ist ein Liposomensystem. Hierbei variiert das Massenverhältnis des mindestens einen Phospholipids zu weiteren, nicht phospholipidischen Substanz zwischen 1:0,001 bis 1:0,1, wobei das erfindungsgemäße Liposomensystem überwiegend solche Liposomen enthält, deren Partikeldurchmesser zwischen 35 nm und 90 nm liegt.

Das erfindungsgemäße System weist eine Reihe von Vorteilen auf. So konnte festgestellt werden, daß das erfindungsgemäß vesikelhaltige System, das ein Liposomensystem ist, auch bei einer Lagerzeit von mehreren Monaten oder Jahren keine Tendenz zeigte, einen Bodensatz oder Ablagerungen an den Gefäßinnenwandungen auszubilden. Desweiteren besitzt das erfindungsgemäße Liposomensystem eine hohe Transparenz und ist nicht, wie bei den bekannten Liposomensystemen, milchig trüb. Aufgrund der fehlenden Ausbildung eines Bodensatzes, der fehlenden Ausbildung von Ablagerungen an Gefäßinnenwandungen und der zuvor wiedergegebenen hohen Transparenz kann das erfindungsgemäße Liposomensystem ohne Schwierigkeiten einer Prüfung auf Fremdpartikeln unterworfen werden, da es hierfür lediglich erforderlich ist, die entsprechende Liposomensdispersion im Durchsichtsverfahren zu überPrüfen.

Die zuvor beschriebenen vorteilhaften Wirkungen des erfindungsgemäßen wäßrigen Liposomensystems werden darauf zurückgeführt, daß es offensichtlich aufgrund der Anwesenheit von bereits sehr geringen Mengen der weiteren, nicht phospholipidischen Substanz (Gallensäure, Gallensäuresalz, Gallensäurederivat und/oder ein Salz eines Gallensäurederivates), die nicht natürlicherweise in phospholipidischen Systemen enthalten sind, zu einem synergistischen Effekt kommt.

Besonders gute Erkenntnisse bezüglich der zuvor beschriebenen Vorteile weist eine erste Ausführungsform des erfindungsgemäßen wäßrigen Liposomensystems auf, bei dem das Massenverhältnis von Phospholipid zu weiteren, nicht phospholipidischen Substanz (Gallensäure, Gallensäuresalz, Gallensäurederivat und/oder ein Salz eines Gallensäurederivates) zwischen 1:0,03 und 1:0,1 liegt.

Wie bereits vorstehend ausgeführt ist, weist die erfindungsgemäße phospholipidische Zusammensetzung mindestens eine weitere, nicht phospholipidische Substanz auf. Hierbei handelt es sich insbesondere um ein Salz, vorzugsweise ein Natriumund/oder ein Ammoniumsalz, der Cholsäure, der Desoxycholsäure, der Ursodesoxycholsäure und/oder der Chenodesoxycholsäure, wobei jedoch auch ein Salz, insbesondere ein Natrium- und/oder ein Ammoniumsalz, eines Gallensäurederivates, vorzugsweise von Glykocholsäure und/oder Taurocholsäure, enthalten sein kann.

Eine weitere, besonders geeignete Ausführungsform des erfindungsgemäßen Liposomensystems sieht vor, daß das erfindungsgemäße Liposomensystem als weitere, nicht phospholipidische Substanz Glykocholsäure, ein Salz von Glykocholsäure und/oder ein Derivat von Glykocholsäure in den eingangs genannten Massenverhältnissen enthält.

Bezüglich des in dem erfindungsgemäßen wäßrigen Liposomensystem enthaltenen Phospholipids ist festzuhalten, daß die Konzentration des mindestens einem Phospholipids zwischen 5 Gew.% und 25 Gew.%, insbesondere zwischen 8 Gew.% und 18 Gew.%, variiert, wobei sich diese Konzentrationsangaben auf das anwendungsfertige Liposomensystem bzw. auf ein Liposomensystem beziehten, wie dieses in den Handel gelangt. Selbstverständlich sind auch abhängig von dem jeweiligen Einsatzgebiet bzw. Anwendungsfall des erfindungsgemäßen Liposomensystems höhere bzw. geringere Phospholipidkonzentrationen als die zuvor angegebenen Werte denkbar.

Eine besonders hohe Stabilität sowie eine besonders gleichmäßige Verteilung der Liposomen bezüglich ihrer Durchmesser ist bei einer anderen Ausführungsform des erfindungsgemäßen Liposomensystems dann gegeben, wenn das erfindungsgemäße Liposomensystem Phosphatidylcholin, insbesondere ein solches Phosphatidylcholin, das aus Sojabohnen gewonnen wird, enthält. Insbesondere dann, wenn das in dem erfindungsgemäßen Liposomensystem vorgesehene Phospholipid zu mindestens 90 Gew.% und vorzugsweise zumindestens 95 Gew.% aus Phosphatidylcholin besteht, besitzt ein derartiges Liposomensystem die eingangs beschriebenen vorteilhaften Eigenschaften in verstärktem Maße. Hinzu kommt noch, daß ein derartiges spezielles Liposomensystem mit wesentlich geringerem Aufwand und somit in etwa der halben Zeit durch Extrusion, Hochdruckspalthomogenisation oder Ultraschallbehandlung auf einen gewünschten mittleren Partikeldurchmesser, der 35 nm und 90 nm liegt, homogen zerkleinert werden kann. Auch läßt sich ein derartiges spezielles Liposomensystem, das überwiegend unilamellare bis bilamellare Liposomen der zuvor genannten Größe aufweist, ohne Probleme steril filtrieren, wobei hierfür vorzugsweise 0,2 µm Filter eingesetzt werden.

Ein weiterer Vorteil des erfindungsgemäßen Liposomensystems ist darin zu sehen, daß das erfindungsgemäße Liposomensystem einen pH-Wert besitzt, der um den Neutralpunkt variiert und der vorzugsweise zwischen 6,0 und 8,0 und insbesondere zwischen 6,2 und 7,4, liegt. Eben so kann das erfindungsgemäß System ein nicht toxisches organisches lösungmittel enthalten.

Das erfindungsgemäße Liposomensystem kann sowohl für pharmazeutische als auch für kosmetische Zwecke verwendet werden, wobei diese Verwendung sowohl als Leer-Liposomensystem als auch in Form eines Liposomensystems, das mit einem entsprechenden kosmetischen oder pharmazeutischen Wirkstoff beladen ist, erfolgen kann.

Wird das erfindungsgemäße Liposomensystem in Form eines Leer-Liposomensystems, d.h. ohne einen entsprechend eingekapselten Wirkstoff, verwendet, so konnte festgestellt werden, daß ein derartiges Leer-System hervorragend zur Behandlung von Atherosklerose, erhöhten Blutfettwerten sowie zur Behandlung von Hepatopathien jeder Genese einsetzbar ist, wobei ein derartiges System vorzugsweise neben Wasser und ggf. einem Alkohol zwischen 5 Gew.% und 20 Gew.% einer Mischung von Phosphatidylcholin der zuvor genannten Reinheit und der weiteren, nicht phospholipidischen Substanz (Gallensäure, ein Gallensäuresalz, ein Gallensäurederivat und/oder ein Salz eines Gallensäurederivates) in dem eingangs genannten Massenverhältnis enthält. Insbesondere wird dann ein derartiges pharmazeutisches Produkt bei seiner Anwendung injiziert.

Wie dies bereits vorstehend erwähnt ist, kann in das erfindungsgemäße Liposomensystem ein kosmetischer oder pharmazeutischer Wirkstoff eingekapselt werden. Hierbei hat sich gezeigt, daß ein derartig eingekapselter Wirkstoff im Vergleich zu der bekannten Aufmachungsform, beispielsweise als Tablette, Dragee o.dgl., eine höhere therapeutische Wirkung, insbesondere Langzeitwirkung, besitzt, so daß die Wirkstoffkonzentration in der Regel entsprechend verringert werden kann, ohne daß hierdurch die therapeutische Wirksamkeit beeinträchtigt wird. Diese Depotwirkung wird darauf zurückgeführt, daß die in dem Liposomensystem eingekapselten Wirkstoffe besonders gleichmäßig über einen längeren Zeitraum während der therapeutischen Anwendung abgegeben werden, so daß unerwünschte Nebenwirkungen nicht auftreten oder zumindestens erheblich reduziert sind. Die Auswahl des jeweiligen Wirkstoffes richtet sich dann nach dem jeweiligen Anwendungsgebiet. So können beispielsweise in dem er-findungsgemäßen Liposomensystem Pentamidin, Pentamidin-Salze, insbesondere Pentamidin-Isethionat und/oder Pentamidin-Derivate gelöst und/oder eingekapselt werden, so daß ein derartiges pharmazeutisches Produkt vorzugsweise zur parenteralen und insbesondere zur pulmonalen Behandlung von pneumocystis-carinii-Pneumonie, der afrikanischen Schlafkrankheit oder von Kala-Azar eingesetzt wird.

Besonders geeignet ist es jedoch, wenn man den pharmazeutischen oder kosmetischen Wirkstoff nicht von Anfang an bei der Herstellung des Liposomensystems einsetzt, sondern den pharmazeutischen bzw. kosmetischen Wirkstoff erst nach der Herstellung des Liposomensystems und insbesondere erst unmittelbar vor der Anwendung zugibt. Dies kann beispielsweise dadurch geschehen, daß man ein wäßriges Leer-Liposomensystem mit dem Wirkstoff, der als Trockensubstanz oder als Lösung, Dispersion, Emulsion oder Suspension in einem nicht toxischen Lösungsmittel vorliegt, vermischt oder ein getrocknetes Liposomensystem in Wasser, das mit dem Wirkstoff versetzt ist, dispergiert. So hergestellte pharmazeutische bzw. kosmetische Produkte weisen dann eine hohe Transparenz auf, so daß sie sehr leicht visuell auf Fremdpartikel überprüft werden können.

Weist hingegen das erfindungsgemäße Liposomensystem als Wirkstoff Doxorubicin x HCl auf, so kann es als entsprechendes pharmazeutisches Produkt zur Behandlung von Krebserkrankungen eingesetzt werden.

Soll hingegen das erfindungsgemäße Liposomensystem zur Behandlung von Viruserkrankungen, insbesondere von Viruserkrankungen der Haut, verwendet werden, so bietet es sich hier an, einen entsprechenden viruziden Wirkstoff, vorzugsweise Rosmarinsäure oder Dextransulfat, in den Vesikeln einzukapseln.

Weiterhin können in dem erfindungsgemäßen Liposomensystem auch die bekannten Wirkstoffe zur Behandlung von Krebs, Aids, Leber- oder Viruserkrankungen eingekapselt werden.

Die vorliegende Erfindung betrifft ferner ein Verfahren zur Herstellung des zuvor beschriebenen Liposomensystems.

Das erfindungsgemäße Verfahren zur Herstellung des Liposomensystems sieht vor, daß man zunächst das mindestens eine Phospholipid mit der mindestens einen weiteren, nicht phospholipidischen Substanz, bei der es sich um Gallensäure, ein Gallensäuresalz, ein Gallensäurederivat und/oder ein Salz eines Gallensäurederivates handelt, in einem Massenverhältnis von 1:0,001 bis 1:0,1, vorzugsweise in einem Massenverhältnis zwischen 1:0,03 bis 1:0,1, in einem organischen Lösungsmittel löst oder dispergiert. Anschließend wird diese Lösung bzw. Dispersion eingeengt und hiernach Wasser unter Ausbildung des Liposomensystems zugegeben.

Das erfindungsgemäße Verfahren weist eine Reihe von Vorteilen auf. Überraschend konnte festgestellt werden, daß sich nach dem erfindungsgemäßen Verfahren besonders einfach, schnell und kostengünstig wäßrige Liposomensysteme herstellen lassen, die eine große Lagerstabilität besitzen, keinen Bodensatz ausbilden, keine Ablagerungen an Gefäßinnenwandungen zeigen und darüber hinaus noch eine hohe Transparenz aufweisen, so daß derartige wäßrige Liposomensysteme besonders einfach und schnell auf Fremdpartikel überprüft werden können. Desweiteren weist das nach dem erfindungsgemäßen Verfahren hergestellte Liposomensystem eine hohe Reproduzierbarkeit bezüglich der Teilchengröße auf, wobei bei dem erfindungsgemäßen Verfahren insbesondere solche unilamellaren bzw. bilamellaren Liposome hergestellt werden, deren Partikeldurchmesser zwischen 35 nm und 90 nm variieren. Auch läßt sich das nach dem erfindungsgemäßen Verfahren hergestellte Liposomensystem steril filtrieren, wobei hierfür vorzugsweise 0,2 µm Filter eingesetzt werden.

Bezüglich des organischen Lösungsmittels, das bei dem erfindungsgemäßen Verfahren zum Dispergieren bzw. Lösen des mindestens einen Phospholipid und der weiteren, nicht phospholipidischen Substanz eingesetzt wird, ist allgemein festzuhalten, daß es sich hierbei um ein nicht toxisches Lösungsmittel handelt, das vorzugsweise gute Löseeigenschaften für das Phospholipid sowie für die nicht phospholipidische Substanz (Gallensäure, Gallensäuresalz, Gallensäurederivat und/oder ein Salz eines Gallensäurederivates) aufweist. Vorzugsweise verwendet man als organisches Lösungsmittel Ethanol, Propanol-1, Propanol-2 oder Benzylalkohol jeweils allein oder in Mischung.

Abhängig von dem jeweils eingesetzten nicht toxischen organischen Lösungsmittel und seiner Mischbarkeit bzw. Verträglichkeit mit Wasser engt man die bei dem erfindungsgemäßen Verfahren anfangs hergestellte Lösung bzw. Dispersion auf unterschiedliche Restvolumina an organischem Lösungsmittel ein. Hierbei variieren diese Restvolumina an organischem Lösungsmittel zwischen 0 Vol.% und 20 Vol.%, vorzugsweise zwischen 0 Vol.% und 10 Vol.%, wobei insbesondere dann, wenn das jeweils eingesetzte organische Lösungsmittel mit Wasser nicht mischbar ist, das anfänglich bei dem erfindungsgemäßen Verfahren eingesetzte organische Lösungsmittel bis zur Trockenheit eingeengt wird.

Um bei dem erfindungsgemäßen Verfahren die Reproduzierbarkeit in bezug auf die Partikelgröße der hiernach hergestellte Liposomensysteme weiter zu verbessern, d.h. um nach dem erfindungsgemäßen Verfahren solche Liposomensysteme herzustellen, die Partikel aufweisen, deren Größe nur geringfügig abweicht, empfiehlt es sich, daß man nach der Zugabe des Wassers das hierbei entstehende Liposomensystem, das aus dem mindestens einem Phospholipid sowie der weiteren, nicht phospholipidischen Substanz (Gallensäure, Gallensäuresalz, Gallensäurederivat und/oder Salz eines Gallensäurederivates) besteht, einer Extrusion, einer Hochdruckspalthomogenisation und/oder einer Ultraschallbehandlung unterwirft. Vorzugsweise wird dabei die Extrusion, die Hochdruckspalthomogenisation und/oder die Ultraschallbehandlung bei einer Temperatur unterhalb von 40 °C, insbesondere bei einer Temperatur zwischen 20 °C und 30 °C, durchgeführt.

Bezüglich der Zeit, die man bei dem erfindungsgemäßen Verfahren für die Extrusion, Hochdruckspalthomogenisation bzw. die Ultraschallbehandlung auswählt, ist allgemein festzuhalten, daß man die zuvor genannten Behandlungen so lange durchführt, bis die dabei gebildeten Liposome den zuvor genannten gewünschten mittleren Durchmesser aufweisen, d.h. die Extrusion, Hochdruckspalthomogenisation bzw. die Ultraschallbehandlung wird so lange durchgeführt, bis die dabei gebildeten Liposome einen mittleren Partikeldurchmesser zwischen 35 nm und 90 nm besitzen.

Eine weitere Ausgestaltung des erfindungsgemäßen Verfahrens sieht vor, daß man das nach dem erfindungsgemäßen Verfahren hergestellte Liposomensystem steril filtriert, vorzugsweise über ein 0,2 µm Filter.

Die nach dem erfindungsgemäßen Verfahren hergestellten wäßrigen Liposomensysteme können direkt anwendungsfertig in entsprechende Ampullen abgefüllt werden. Eine besonders geeignete Ausführungsvariante des erfindungsgemäßen Verfahrens sieht vor, daß man das durch die Zugabe des Wassers gebildete Liposomensystem nach Zusatz eines geeigneten Hilfsstoffes, insbesondere eines Kohlehydrates, schonend trocknet, wobei sich für eine derartige schonende Trocknung vorzugsweise die Gefriertrocknung anbietet. Somit entsteht bei dieser Ausführungsform des erfindungsgemäßen Verfahrens ein pulverartiges Liposomensystem, das durch Zugabe einer geeigneten Menge Wasser wieder die dann direkt anwendungsbereiten erwünschten Vesikel ausbildet, ohne daß es hierbei erforderlich ist, das durch Zusatz von Wasser entstehende wäßrige Liposomensystem aufwendig zu rühren oder beispielsweise einer Hochdruckspalthomogenisation oder Ultraschallbehandlung zu unterwerfen.

Wie bereits vorstehend beim erfindungsgemäßen vesikelhaltigen wäßrigen System ausgeführt ist, wird bei dem erfindungsgemäßen Verfahren vorzugsweise ein Soja-Phospholipid und insbesondere ein solches Soja-Phospholipid ausgewählt, das eine hohe Konzentration an Phosphatidylcholin, vorzugsweise mindestens 90 Gew.% Phosphatidylcholin, enthält.

Um die zuvor beschriebenen Ausführungsformen des erfindungsgemäßen wäßringen Systems, die einen der zuvor genannten pharmazeutischen oder kosmetischen Wirkstoff aufweisen, herzustellen, bestehen zwei Möglichkeiten.

So sieht die erste Möglichkeit vor, daß man den Wirkstoff zusammen mit dem Phospholipid und der weiteren, nicht phospholipidischen Substanz, bei der es sich um Gallensäure, eine Gallensäuresalz, ein Gallensäurederivat und/oder ein Salz eines Gallensäurederivates handelt, direkt zu Beginn des erfindungsgemäßen Verfahrens in dem eingangs genannten Massenverhältnis dem eingesetzte organische Lösungsmittel zugibt.

Bei einer Abwandlung dieser zuvor beschriebenen Verfahrensvariante setzt man ein Phospholipid ein, das mit dem gewünschten Wirkstoff beladen ist. Um dies zu erreichen, tränkt man das ausgewählte Phospholipid mit dem in einem nicht wäßrigen Lösungsmittel gelösten, dispergierten bzw. emulgierten Wirkstoff und führt dann eine schonende Trocknung des so mit dem Wirkstoff beladenen Phospholipids durch. Hiernach wird das entsprechend mit dem Wirkstoff beladene Phospholipid zusammen mit der weiteren, nicht phospholipidischen Substanz, in einem organischen Lösungsmittel, das ggf. von dem für die Beladung eingesetzten Lösungsmittel unterschiedlich ist, gelöst. Hiernach wird, wie dies vorstehend bei dem erfindungsgemäßen Verfahren beschrieben ist, das organische Lösungsmittel eingeengt, so daß man danach das Wasser zugibt, wodurch ein Liposomensystem ausgebildet wird, das einen Wirkstoff einkapselt. Diese Verfahrensvariante bietet sich insbesondere für solche Fälle an, bei denen der Wirkstoff bezüglich seiner Lagerung stabil ist.

Die zweite Möglichkeit, die insbesondere dann bevorzugt wird, wenn der Wirkstoff besser in Wasser als in dem organischen Lösungsmittel löslich ist, sieht vor, daß man zunächst in der vorstehend beschriebenen Weise das mindestens eine Phospholipid mit der mindestens einen weiteren, nicht phospholipidischen Substanz in dem ausgewählten organischen Lösungsmittel löst bzw. dispergiert. Nach dem Einengen der Lösung bzw. Dispersion gibt man den Wirkstoff zusammen mit dem Wasser zu, wobei vorzugsweise der Wirkstoff zuvor im Wasser aufgenommen wurde.

Eine Abwandlung der zuvor beschriebenen Verfahrensvariante, die insbesondere dann angewendet wird, wenn der Wirkstoff nur eine begrenzte Haltbarkeit besitzt, geht von einem pulverförmigen, getrockneten Liposomensystem aus. Hierbei wird bei der Redispergierung zusammen mit dem dabei eingesetzten Wasser der Wirkstoff zugesetzt, so daß somit unmittelbar vor der Anwendung eines derartiges Produktes erst der Wirkstoff in das Liposomensystem eingekapselt wird, so daß er entsprechend gegenüber Alterung geschützt ist.

Um unerwünschte Nebenwirkungen auszuschließen, wird das erfindungsgemäße Verfahren vorzugsweise unter Schutzgas (Inertgas) durchgeführt.

Wie bereits vorstehend beim erfindungsgemäßen wäßrigen System ausgeführt ist, weist dieses vorzugsweise eine Phospholipid-Konzentration zwischen 5 Gew.% und 25 Gew.%, insbesondere zwischen 8 Gew.% und 18 Gew.%, auf, wobei abhängig von den jeweiligen Anwendungsfällen bzw. Einsatzgebieten diese Konzentrationswerte über- als auch unterschritten werden können, sofern sichergestellt ist, daß das eingangs genannte Massenverhältnis von Phospholipid zur weiteren, nicht phospholipidischen Substanz eingehalten wird. Dementsprechend setzt man bei dem erfindungsgemäßen Verfahren eine entsprechende Menge des mindestens einen Phospholipids ein und wählt das Massenverhältnis von Phospholipid zu der mindestens einen nicht phospholipidischen Substanz im Sinne der vorstehenden Ausführungen (1:0,001 bis 1:0,1) aus.

Vorteilhafte Weiterbildungen des erfindungsgemäßen Vesikelhaltigen wäßrigen Systems und des erfindungsgemäßen Verfahrens sind in den Unteransprüchen angegeben.

Das erfindungsgemäße Verfahren wird nachfolgend anhand von Ausführungsbeispielen näher erläutert.

Zur Herstellung von verschiedenen wäßrigen Liposomensystemen wurden die nachfolgend wiedergegebenen Zusammensetzungen I bis VIII in 500 ml Ethylalkohol gelöst. Anschließend wurde das Lösungsmittel unter Vakuum bis zur Trockenheit eingeengt. Der hierbei anfallende Rückstand wurde in 1.000 ml Wasser dispergiert und danach mittels Hochdruckspalthomogenisation auf einen mittleren Partikeldurchmesser zwischen 30 nm und 100 nm gebracht. Hiernach wurde das entstandene Liposomensystem unter sterilen Bedingungen über ein 0,2 µm Filter filtriert und unter sterilen Bedingungen in Ampullen abgefüllt.

Zur Vermeidung von unerwünschten Oxidationsreaktionen wurde das zuvor beschriebene Herstellungsverfahren insgesamt unter Inertgas (Stickstoff) durchgeführt.

Für das zuvor beschriebene Herstellungsverfahren wurden die nachfolgend wiedergegebenen Zusammensetzungen I - VIII eingesetzt, wobei das dort aufgeführte Phosphatidylcholin ein hochreines Soja-Phosphatidylcholin war, das mehr als 90 Gew.% Phosphatidylcholin enthält.

### Zusammensetzung I

100 g Phosphatidylcholin

### Zusammensetzung II

99,5 g Phosphatidylcholin
0,5 g Natriumsalz des Desoxycholsäure

### Zusammensetzung III

99,5 g Phosphatidylcholin
0,5 g Natriumsalz der Chenodesoxycholsäure

### Zusammensetzung IV

99,5 g Phosphatidylcholin
0,5 g Natriumsalz der Cholsäure

### Zusammensetzung V

95 g Phosphatidylcholin
5 g Natriumsalz der Glykocholsäure

### Zusammensetzung VI

99 g Phosphatidylcholin
1 g Natriumsalz der Glykocholsäure

### Zusammensetzung VII

99,5 g Phosphatidylcholin
0,5 g Natriumsalz der Glykocholsäure

### Zusammensetzung VIII

99,7 g Phosphatidylcholin
0,3 g Natriumsalz der Glykocholsäure

Das wäßrige Liposomensystem, das unter Verwendung der Zusammensetzung I hergestellt wurde, unterschied sich deutlich in seinen Eigenschaften von den wäßrigen Liposomensystemen, die unter Verwendung der Zusammensetzungen II bis VIII erstellt wurden, wie dies der nachfolgenden Tabelle 1 zu entnehmen ist.

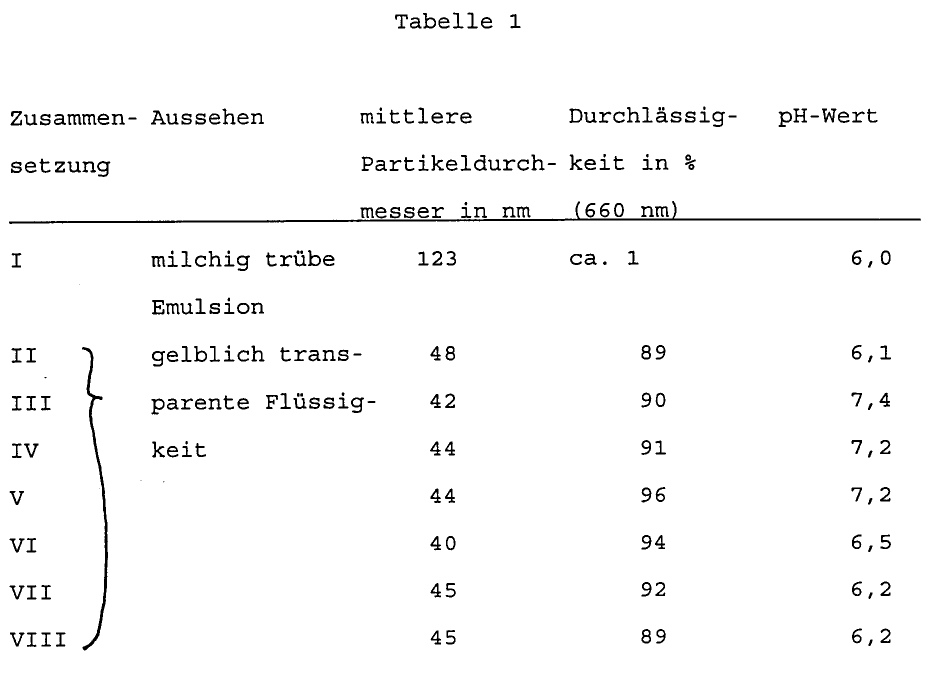

Desweiteren ist festzuhalten, daß nur die wäßrigen LiposomenSysteme gemäß der Zusammensetzungen II bis VIII einwandfrei mit hoher Ausbeute über ein 0,2 µm Filter steril filtrierbar waren, während das wäßrige Liposomensystem, das aus der Zusammensetzung I erstellt wurde, nicht filtrierbar war.

## Patentansprüche

1. Vesikelhaltiges wäßriges System, das mindestens ein Phospholipid sowie mindestens eine weitere, nicht phospholipidische Substanz enthält, bei der es sich um Gallensäure, ein Gallensäuresalz, ein Gallensäurederivat und/oder ein Salz eines Gallensäurederivates handelt, dadurch gekennzeichnet, daß das vesikelhaltige wäßrige System ein Liposomensystem ist, daß das Massenverhältnis von Phospholipid zur weiteren, nicht phospholipidischen Substanz zwischen 1:0,001 bis 1:0,1 variiert und daß das Liposomensystem überwiegend Liposome mit einem Partikeldurchmesser zwischen 35 nm und 90 nm aufweist.

2. Liposomensystem nach Anspruch 1, dadurch gekennzeichnet, daß das Massenverhältnis von Phospholipid zur weiteren, nicht phospholipidischen Substanz zwischen 1:0,03 und 1:0,1 liegt.

3. Liposomensystem nach Anspruch 1 oder 2, dadurch gekennzeichnet, daß es ein Salz, vorzugsweise ein Natrium- und/oder ein Ammoniumsalz, der Cholsäure, der Desoxycholsäure, der Ursodesoxycholsäure und/oder der Chenodesoxycholsäure aufweist.

4. Liposomensystem nach einem der vorangehenden Ansprüche, dadurch gekennzeichnet, daß es als Salz des Gallensäurederivates ein Salz von Glykocholsäure und/oder Taurocholsäure aufweist.

5. Liposomensystem nach einem der vorangehenden Ansprüche, dadurch gekennzeichnet, daß es Glykocholsäure enthält.

6. Liposomensystem nach einem der vorangehenden Ansprüche, dadurch gekennzeichnet, daß es das Phospholipid in einer Konzentration zwischen 5 Gew.% und 25 Gew.%, insbesondere in einer Konzentration zwischen 8 Gew.% und 18 Gew.%, jeweils bezogen auf das anwendungsfertige Liposomensystem aufweist.

7. Liposomensystem nach einem der vorangehenden Ansprüche, dadurch gekennzeichnet, daß es überwiegend Phosphatidylcholin als Phospholipid enthält.

8. Liposomensystem nach Anspruch 7, dadurch gekennzeichnet, daß das Phospholipid zu mindestens 90 Gew.% aus Phosphatidylcholin besteht.

9. Liposomensystem nach einem der vorangehenden Ansprüche, dadurch gekennzeichnet, daß das Phospholipid ein Sojaphospholipid ist.

10. Liposomensystem nach einem der vorangehenden Ansprüche, dadurch gekennzeichnet, daß das Liposomensystem einen pH-Wert zwischen 6,0 und 8,0 aufweist.

11. Liposomensystem nach einem der vorangehenden Ansprüche, dadurch gekennzeichnet, daß das Liposomensystem ein nicht toxisches organisches Lösungsmittel enthält.

12. Liposomensystem nach einem der vorangehenden Ansprüche, dadurch gekennzeichnet, daß das Liposomensystem mindestens eine pharmazeutisch wirksame Substanz beinhaltet.

13. Liposomensystem nach Anspruch 12, dadurch gekennzeichnet, daß die pharmazeutisch wirksame Substanz ein Wirkstoff zur Behandlung von Krebs, Aids, Lebererkrankungen, Viruserkrankungen oder Pneumocystis-carinii-Pneumonie ist.

14. Verfahren zur Herstellung des Liposomensystems nach einem der Ansprüche 1 bis 13, dadurch gekennzeichnet, daß man zunächst das Phospholipid mit der mindestens einen weiteren, nicht phospholipidischen Substanz in einem Massenverhältnis von 1:0,001 bis 1:0,1, vorzugsweise in einem Massenverhältnis von 1:0,03 bis 1:0,1, in einem organischen Lösungsmittel löst oder dispergiert, anschließend die Lösung bzw. Dispersion einengt und hiernach Wasser unter Ausbildung des Liposomensystems zugibt.

15. Verfahren nach Anspruch 14, dadurch gekennzeichnet, daß man als organisches Lösungsmittel Ethanol, Propanol-1, Propanol-2 und/oder Benzylalkohol verwendet.

16. Verfahren nach Anspruch 14 oder 15, dadurch gekennzeichnet, daß man die Lösung bzw. Dispersion auf ein Restvolumen an organischem Lösungsmittel von 0 Vol.% bis 20 Vol.%, vorzugsweise von 0 Vol.% bis 10 Vol.%, einengt.

17. Verfahren nach einem der Ansprüche 14 bis 16, dadurch gekennzeichnet, daß man nach der Zugabe des Wassers das hierbei entstehende Liposomensystem einer Extrusion, einer Hochdruckspalthomogenisation und/oder einer Ultraschallbehandlung unterwirft.

18. Verfahren nach Anspruch 17, dadurch gekennzeichnet, daß man die Extrusion, die Hochdruckspalthomogenisation und/oder die Ultraschallbehandlung bei einer Temperatur unterhalb von 40 °C durchführt.

19. Verfahren nach Anspruch 17 oder 18, dadurch gekennzeichnet, daß man die Extrusion, die Hochdruckspalthomogenisation bzw. die Ultraschallbehandlung so lange durchführt, bis die dabei gebildeten Liposome einen mittleren Durchmesser zwischen 35 nm und 90 nm aufweisen.

20. Verfahren nach einem der Ansprüche 14 bis 19, dadurch gekennzeichnet, daß man das Liposomensystem über ein 0,2 µm Filter filtriert.

21. Verfahren nach einem der Ansprüche 14 bis 20, dadurch gekennzeichnet, daß man das durch die Zugabe des Wasser gebildete Liposomensystem nach Zusatz eines geeigneten Hilfsstoffes, insbesondere eines Kohlehydrates, schonend trocknet, insbesondere gefriergetrocknet.

22. Verfahren zur Herstellung eines mit einem Wirkstoff versehenen Liposomensystems nach einem der Ansprüche 13 bis 21, dadurch gekennzeichnet, daß man den Wirkstoff zusammen mit dem Phospholipid und der mindestens einen weiteren, nicht phospholipidischen Substanz in dem organischen Lösungsmittel löst, emulgiert oder dispergiert.

23. Verfahren zur Herstellung eines mit einem Wirkstoff versehenen Liposomensystems nach Anspruch 21, dadurch gekennzeichnet,daß man das getrocknete Liposomensystem in Wasser, dem mindestens eine pharmazeutisch wirksame Substanz zugesetzt ist, aufnimmt.

24. Verfahren nach einem der Ansprüche 14 bis 23, dadurch gekennzeichnet, daß man das Phospholipid in einer Konzentration zwischen 5 Gew.% und 25 Gew.%, vorzugsweise in einer Konzentration zwischen 8 Gew.% und 18 Gew.%, jeweils bezogen auf das anwendungsfertige Liposomensystem in dem organischen Lösungsmittel löst bzw. dispergiert.

## Claims

1. An aqueous vesicles containing system comprising at least one phospholipid and a further non-phospholipidic substance, said substance being a bile acid, a salt of a bile acid, a bile acid derivative and/or a salt of a bile acid derivative, wherein said aqueous vesicles containing system is a liposome system, wherein the mass ratio of the phospholipid to said further non-phospholipidic substance varies between 1:0,001 to 1:0,1 and wherein said liposome system predominantly contains liposomes having a particle diameter between 35 nm and 90 nm.

2. The liposome system according to claim 1, wherein the mass ratio of phospholipid to said further non-phospholipidic substance lies between 1:0,03 and 1:0,1.

3. The liposome system according to claim 1 or 2, wherein said liposome system contains a salt, preferably a sodium- and/or an ammonium salt, of cholic acid, of deoxycholic acid, of ursodeoxycholic acid and/or chenodeoxycholic acid.

4. The liposome system according to one of the preceding claims, wherein said liposome system contains as a salt of the bile acid derivative a salt of glycocholic acid and/or taurocholic acid.

5. The liposome system according to one of the preceding claims, wherein said liposome system contains glycocholic acid.

6. The liposome system according to one of the preceding claims, wherein the phospholipid is present in a concentration of between 5 % by weight and 25 % by weight, in particular in a concentration of between 8 % by weight and 18 % by weight, with respect to the ready to use liposome system.

7. The liposome system according to one of the preceding claims, wherein said phospholipid is predominantly phosphatidylcholine.

8. The liposome system according to claim 7, wherein said phospholipid comprises at least 90 % by weight phosphatidylcholine.

9. The liposome system according to one of the preceding claims, wherein said phospholipid is a soybean phospholipid.

10. The liposome system according to one of the preceding claims, wherein said liposome system possesses a pH value between 6,0 and 8,0.

11. The liposome system according to one of the preceding claims, wherein said liposome system contains a non-toxic organic solvent.

12. The liposome system according to one of the preceding claims, wherein said liposome system contains at least one pharmaceutically active ingredient.

13. The liposome system according to claim 12, wherein said pharmaceutically active ingredient is a drug for the treatment of cancer, aids, liver diseases, viral diseases or Pneumocystis carinii pneumonia.

14. A method for the preparation of the liposome system according to one of the claims 1 to 13, wherein said phospholipid is dissolved or dispersed with said at least one further non-phospholipidic substance in a mass ratio of 1:0,001 to 1:0,1 preferably in a mass ratio of 1:0,03 to 1:0,1 in an organic solvent, hereafter said organic solvent is evaporated partially or completely from said solution or dispersion and water is added to form the liposome system.

15. The method according to claim 14, wherein said organic solvent is selected from the group consisting of ethanol, 1-propanol, 2-propanol and benzyl alcohol or combinations thereof.

16. The method according to claim 14 or 15, wherein said organic solvent is evaporated partly or completely from the solution or the dispersion to a remaining volume of organic solvent between 0 volume % and 20 volume %, preferably between 0 volume % and 10 volume %.

17. The method according to one of the claims 14 to 16, wherein said liposome system formed after the addition of water is subjected to an extrusion, a high pressure split homogenisation and/or an ultrasound treatment.

18. The method according to claim 17, wherein said extrusion, said high pressure split homogenisation and/or said ultrasound treatment are performed at a temperature lower than 40 °C.

19. The method according to claim 17 or 18, wherein said extrusion, said high pressure split homogenisation and/or said ultrasound treatment is performed for a sufficiently long time, to obtain liposomes with a mean diameter between 35 nm and 90 nm.

20. The method according to one of the claims 14 to 19, wherein said liposome system is filtered through a 0,2 µm filter.

21. The method according to one of the claims 14 to 20, wherein said liposome system formed after the addition of water is carefully brought to dryness, in particular by lyophilisation, after the addition of a suitable additive, in particular a carbohydrate.

22. A method to prepare a liposome system according to one of the claims 13 to 21 containing an active ingredient, wherein said active ingredient is dissolved, emulsified or dispersed in said organic solvent together with said phospholipid and said at least one further non-phospholipidic substance.

23. The method to prepare a liposome system containing a active ingredient according to claim 21, wherein said water which is added to said dried liposome system contains said pharmaceutically active ingredient.

24. The method according to one of the claims 14 to 23, wherein said phospholipid is dissolved or dispersed in said organic solvent in a concentration of between 5 % by weight and 25 % by weight, preferably in a concentration of between 8 % by weight and 18 % by weight, relative to the ready to use liposome system.

## Revendications

1. Système aqueux contenant des vésicules, qui comporte au moins un phospholipide ainsi qu'au moins une autre substance non phospholipidique, pour laquelle il s'agit d'acide biliaire, d'un sel d'acide biliaire, et d'un dérivé d'acide biliaire et/ou d'un sel d'un dérivé d'acide biliaire, caractérisé en ce que le système aqueux contenant des vésicules est un système de liposomes, en ce que le rapport en masse entre le phospholipide et l'autre substance non phospholipidique varie entre 1/0,001 et 1/0,1 et en ce que le système de liposomes présente principalement des liposomes ayant un diamètre des particules compris entre 35 nm et 90 nm.

2. Système de liposomes suivant la revendication 1, caractérisé en ce que le rapport en masse entre le phospholipide et l'autre substance non phospholipidique est compris entre 1/0,03 et 1/0,1.

3. Système de liposomes suivant l'une des revendications 1 et 2, caractérisé en ce qu'il présente un sel, de préférence un sel de sodium et/ou d'ammonium, de l'acide cholique, de l'acide désoxycholique, de l'acide ursodésoxycholique et/ou de l'acide chénodésoxycholique.

4. Système de liposomes suivant l'une des revendications précédentes, caractérisé en ce qu'il présente, comme sel du dérivé d'acide biliaire, un sel d'acide glycocholique et/ou d'acide taurocholique.

5. Système de liposomes suivant l'une des revendications précédentes, caractérisé en ce qu'il contient de l'acide glycocholique.

6. Système de liposomes suivant l'une des revendications précédentes, caractérisé en ce qu'il comporte le phospholipide en une concentration entre 5% en poids et 25% en poids, en particulier en une concentration entre 8% en poids et 18% en poids, respectivement par rapport au système de liposomes prêt à l'usage.

7. Système de liposomes suivant l'une des revendications précédentes, caractérisé en ce qu'il contient de manière prépondérante de la phosphatidylcholine comme phospholipide.

8. Système de liposomes suivant la revendication 7, caractérisé en ce que le phospholipide est constitué d'au moins 90% en poids de phosphatidylcholine.

9. Système de liposomes suivant l'une des revendications précédentes, caractérisé en ce que le phospholipide est un phospholipide de soja.

10. Système de liposomes suivant l'une des revendications précédentes, caractérisé en ce que le système de liposomes présente une valeur de pH comprise entre 6,0 et 8,0.

11. Système de liposomes suivant l'une des revendications précédentes, caractérisé en ce que le système de liposomes contient un solvant organique non toxique.

12. Système de liposomes suivant l'une des revendications précédentes, caractérisé en ce que le système de liposomes contient au moins une substance pharmaceutiquement active.

13. Système de liposomes suivant la revendication 12, caractérisé en ce que la substance pharmaceutiquement active est une substance active pour le traitement du cancer, du sida, des maladies du foie, des maladies virales ou de la pneumonie Pneumocystis carinii.

14. Procédé de préparation du système de liposomes suivant l'une des revendications 1 à 13, caractérisé en ce qu'on dissout ou disperse tout d'abord le phospholipide dans un solvant organique avec la au moins une autre substance non phospholipidique, dans un rapport en masse de 1/0,001 à 1/0,1, de préférence dans un rapport en masse de 1/0,03 à 1/0,1, puis on concentre la solution ou dispersion et ensuite on ajoute de l'eau avec réalisation du système de liposomes.

15. Procédé suivant la revendication 14, caractérisé en ce que, comme solvant organique, on utilise de l'éthanol, du propanol-1, du propanol-2 et/ou de l'alcool benzylique.

16. Procédé suivant l'une des revendications 14 et 15, caractérisé en ce qu'on concentre la solution ou dispersion à un volume résiduaire en solvant organique de 0% en volume à 20% en volume, de préférence de 0% en volume à 10% en volume.

17. Procédé suivant l'une des revendications 14 à 16, caractérisé en ce que, après l'addition de l'eau, on soumet le système de liposomes alors formé à une extrusion, à une homogénéisation par fragmentation sous haute pression et/ou à un traitement aux ultrasons.

18. Procédé suivant la revendication 17, caractérisé en ce qu'on effectue l'extrusion, l'homogénéisation par fragmentation sous une haute pression et/ou le traitement aux ultrasons à une température inférieure à 40°C.

19. Procédé suivant l'une des revendications 17 et 18, caractérisé en ce qu'on effectue l'extrusion, l'homogénéisation par fragmentation sous haute pression ou le traitement aux ultrasons suffisamment longtemps pour que les liposomes alors formés présentent un diamètre moyen compris entre 35 nm et 90 nm.

20. Procédé suivant l'une des revendications 14 à 19, caractérisé en ce qu'on filtre le système de liposomes sur un filtre de 0,2 µm.

21. Procédé suivant l'une des revendications 14 à 20, caractérisé en ce qu'on sèche de manière ménagée, en particulier lyophilise, le système de liposomes formé par l'addition de l'eau, après addition d'une substance auxiliaire appropriée, en particulier d'un hydrate de carbone.

22. Procédé de préparation d'un système de liposomes pourvu d'une substance active, suivant une des revendications 13 à 21, caractérisé en ce qu'on dissout, émulsionne ou disperse dans le solvant organique la substance active conjointement au phospholipide et à la au moins une autre substance non phospholipidique.

23. Procédé de préparation d'un système de liposomes pourvu d'une substance active, suivant la revendication 21, caractérisé en ce qu'on reprend le système de liposomes séché dans de l'eau, à laquelle on a ajouté au moins une substance pharmaceutiquement active.

24. Procédé suivant l'une des revendications 14 à 23, caractérisé en ce qu'on dissout ou disperse dans le solvant organique le phospholipide en une concentration comprise entre 5% en poids et 25% en poids, de préférence en une concentration entre 8% en poids et 18% en poids, respectivement par rapport au système de liposomes prêt à l'usage.
